(19) Europäisches Patentamt
European Patent Office
Office européen des brevets

(11) Publication number: **0 100 644**
**B2**

(12) **NEW EUROPEAN PATENT SPECIFICATION**

(45) Date of publication of the new patent specification: **24.10.90**

(51) Int. Cl.[5]: **A 61 K 7/08**

(21) Application number: **83304325.0**

(22) Date of filing: **27.07.83**

(54) **Shampoo compositions and method of cleaning hair therewith.**

(30) Priority: **30.07.82 US 403910**
**22.06.83 US 506917**

(43) Date of publication of application:
**15.02.84 Bulletin 84/07**

(45) Publication of the grant of the patent:
**29.04.87 Bulletin 87/18**

(45) Mention of the opposition decision:
**24.10.90 Bulletin 90/43**

(84) Designated Contracting States:
**AT BE CH DE FR IT LI LU NL SE**

(56) References cited:
EP-A-0 056 595
US-A-2 892 756
US-A-2 950 255
US-A-3 427 251
US-A-3 990 991
US-A-4 110 263
US-A-4 321 156
US-A-4 329 335

**H. JANISTYN: "HANDBUCH DER KOSMETIKA UND RIECHSTOFFE", vol. 3, "Die Körperpflegemittel", 2nd edition, 1973, Dr. Alfred Hüthig Verlag, Heidelberg, DE.**

(73) Proprietor: **THE PROCTER & GAMBLE COMPANY**
**301 East Sixth Street**
**Cincinnati Ohio 45202 (US)**

(72) Inventor: **Steuri, Christian**
**3, St. James Court**
**Fairfield Ohio 45014 (US)**

(74) Representative: **Brooks, Maxim Courtney et al**
**Procter & Gamble (NTC) Limited Whitley Road**
**Longbenton**
**Newcastle-upon-Tyne NE12 9TS (GB)**

(56) References cited:
**CFTA COSMETIC INGREDIENT DICTIONARY, 3rd edition, 1982, The Cosmetic, Toiletry and Fragrance Association, Inc., Washington, D.C., USA**
**Seifen, Oele, Fette, Wachse 100 1974,p.41**

**EP 0 100 644 B2**

## Description

The desire to develop products which simultaneously clean and condition hair has long been present. While the desire has long been present, developing such products has presented innumerable problems. Generally the agents which condition hair best are cationic with one or more long fatty hydrocarbon chains. Hair being negatively charged will allow for the cationic portion to attach to the hair while the long fatty chain(s) provide for ease of combing and hair conditioning.

Cationic materials generally cannot be used with good cleaning anionic surfactants and still deliver good hair condition. This meant that other surfactants such as nonionics, amphoterics and zwitterionics were examined by workers in the field. Many of these efforts are reflected in patents issued in the conditioning shampoo area.

US—A—3,849,348 discloses conditioning shampoos containing betaine, cationic and amine oxide surfactants. US—A—3,697,452 discloses similar shampoo compositions. US—A—3,755,559 discloses shampoos containing a tertiary amine oxide, a higher alkyl betaine and a soap. US—A—3,822,312 discloses shampoos containing a quaternary ammonium salt, a betaine and an additional additive. US—A—3,990,991 discloses shampoos containing amphoteric surfactants and quaternary ammonium compounds. US—A—4,080,310 discloses shampoos containing an amphoteric surfactant, a cationic resin and having a pH as low as 3. US—A—4,132,679 discloses shampoos containing a phosphoric acid ester salt and a betaine. US—A—4,231,903 discloses shampoos containing a mixture of an amido betaine and a phosphobetaine. US—A—4,247,548 discloses a conditioning shampoo containing a betaine, a polypropoxylated quaternary ammonium chloride surfactant and gum arabic. US—A—4,294,728 discloses shampoos containing a cationic, amphoteric or zwitterionic surfactant and a diol. US—A—4,329,335 discloses a shampoo composition containing a betaine, an amine oxide and a polymerized quaternary compound. US—A—4,181,634 discloses shampoos containing a bisquarternary compound. EP—A—56595 relates to a hair conditioning composition comprising betaine and an aliphatic acid. Finally, H. Janistyn, "Handbuch der Kosmetika and Reichstoffe, Vol. III, pages 278—9 (1973)" discloses conditioning shampoos incorporating betaines and quaternary ammonium compounds.

While the above described references disclose compositions containing components of the type used in the present compositions, they do not teach or suggest totally satisfactory answers to the questions of good cleaning, conditioning and stability (freeze thaw). It is believed that good cleaning with quaternary compounds is in part dependent on limiting the reacting of the quaternary with the fatty acids in sebum.

In addition the references fail to teach or suggest combining betaine surfactants of the type disclosed herein with quaternary ammonium compounds in compositions having a pH in the range of from 2 to 4.

It is, therefore, an object of the present invention to provide hair conditioning shampoo compositions which provide good cleaning and conditioning. The good cleaning relates to improved sebum emulsification as well as good lather.

It is a further object of the present invention to provide shampoo compositions containing particular betaine surfactants, and quaternary ammonium compounds and having pH in the range of from 2 to 4.

These and other objects will become more apparent from the detailed description which follows.

Unless otherwise indicated, all percentages and ratios herein are by weight.

Disclosure of the invention

The present invention relates to a shampoo composition free of anionic surfactant and comprising:

(A) from 0.5% to 10% of an ammonium compound having the formula

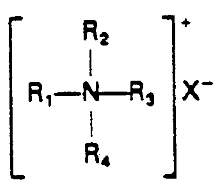

or

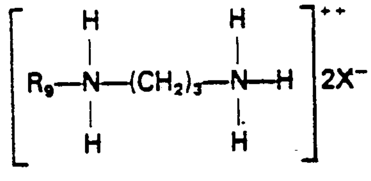

wherein $R_1$ is hydrogen, an aliphatic group of from 1 to 22 carbon atoms, or an aryl or alkaryl group having 6 to 20 carbon atoms; $R_2$ is an aliphatic group having from 12 to 22 carbon atoms; $R_3$ and $R_4$ are each alkyl groups having from 1 to 3 carbon atoms; $R_9$ is an aliphatic group having 16 to 22 carbon atoms; and X is an

anion selected from halogen, acetate, phosphate, nitrate and methyl sulfate radicals, or an imidazolinium salt having the formula

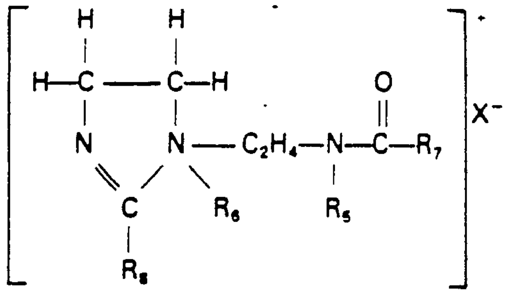

wherein $R_6$ is an alkyl group containing from 1 to 4 carbon atoms; $R_5$ is an alkyl group containing from 1 to 4 carbon atoms or a hydrogen atom; $R_8$ is an alkyl group containing from 1 to 22 carbon atoms, or a hydrogen atom; $R_7$ is an alkyl group containing from 8 to 22 carbon atoms; and X is anion as defined above,

(B) from 5% to 70% of a $C_8$—$C_{18}$ alkylamido betaine surfactant; and

(C) water,

characterized in that the pH of said composition is in the range of 2.0 to 4.0, being maintained within said range by means of an acid buffering agent.

Detailed description of the invention

The essential as well as optional components of the present invention are described in detail below.

Surfactant

The essential surfactants used in the compositions of the present invention are higher ($C_8$—$C_{18}$) alkylamido betaines.

The betaines may be represented by the following structural formula:

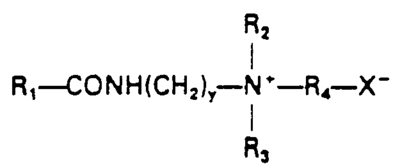

wherein $R_1$ is a long chain alkyl radical having from 10 to 18 carbon atoms, $R_2$ and $R_3$ are each alkyl radicals having from 1 to 3 carbon atoms, $R_4$ is an alkylene or hydroxy alkylene radical having from 1 to 4 carbon atoms, y is an integer from 1 to 4, and X is a carboxylate radical, $R_1$ may be a mixture of long chain alkyl radicals and may contain one or more intermediate linkages or non-functional substituents such as hydroxyl or halogen radicals which do not affect the hydrophobic character of the radical. Examples of betaines useful herein include cocoamidopropyldimethylcarboxymethyl betaine, laurylamidopropyldimethylcarboxymethyl betaine among many others. In many instances the dimethylcarboxymethyl part of the designation is not included.

The amount of surfactant is from 5% to 70% preferably from 10% to 25%.

Ammonium or imidazolinium compound

Preferred ammonium salts are the dialkyldimethylammonium chlorides, wherein the alkyl groups have from 12 to 22 carbon atoms and are derived from long chain fatty acids, such as tallow or hydrogenated tallow. The term "tallow" refers to fatty alkyl groups derived from tallow fatty acids. Such fatty acids give rise to compounds wherein $R_1$ and $R_2$ have predominately from 16 to 18 carbon atoms.

Representative examples of quaternary ammonium salts useful in this invention include ditallowdimethylammonium chloride, ditallowdimethylammonium methyl sulfate, dihexadecyldimethylammonium chloride, di(hydrogenated tallow) dimethylammonium chloride, dioctadecylmethylammonium chloride, dieicosyldimethylammonium chloride; didocosyldimethylammonium chloride, di(hydrogenated tallow)dimethylammonium acetate, dihexadecyldiethylammonium chloride, dihexadecyldimethylammonium acetate, ditallowdipropylammonium phosphate, ditallowdimethylammonium nitrate, di(coconut-alkyl)dimethylammonium chloride; and cetyltrimethylammonium chloride.

Tallow propanediamine hydrochloride is an example of an ammonium salt of the formula

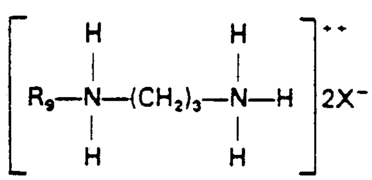

wherein $R_9$ is an aliphatic group having 16 to 22 carbon atoms and X is an anion as above defined.

In the imidazolinium salts, $R_6$ is preferably an alkyl group containing 1 to 2 carbon atoms, $R_8$ is preferably an alkyl group containing at least 15 carbon atoms, $R_7$ is preferably an alkyl group containing 15 carbon atoms and X is preferably chloride.

Particularly preferred are those imidazolinium salts in which both $R_7$ and $R_8$ are alkyl of from 12 to 22 chloride; 1 - methyl - 1 - [(stearoylamide)ethyl] - 2 - heptadecyl - 4,5 - dihydroimidazolinium chloride; 1 - methyl - 1 - [(palmitoylamide]ethyl] - 2 - octadecyl - 4,5 - dihydroimidazolinium chloride; and 1 - methyl - 1 - [(tallowamide] - ethyl] - 2 - tallow - imidazolinium methyl sulfate.

The ammonium or imidazolinium salt is present at a level of from 0.5% to 10%, preferably from 1% to 6%.

pH Adjustment

The compositions of the present invention have pH in the range of from 2 to 4, preferably from 2.9 to 3.8. The compositions are adjusted to this pH range with an acid buffer. The buffering capability is necessary since the pH of the diluted product on hair should be within the range given. Suitable buffer solutions can be prepared, using for example agents such as citric acid, phosphonic acid, phthalic acid, glycine or mixtures thereof. In each case the proper buffering capacity is obtained by adjusting the final pH of the compositions to within the pH range indicated above. This may be done by using a strong acid or a strong base (e.g., HCl or NaOH) as may be needed. The most preferred agent is citric acid. The amount of buffer employed in the present compositions depends on the particular acid chosen but is generally from 0.3% to 6%, preferably from 1% to 5%.

Water

Water is the last essential component of the present invention and forms the remainder of the composition. It is generally present at a level of at least 20%, preferably from 65% to 80%.

Optional components

The shampoos herein can contain a variety of non-essential optional components suitable for rendering such compositions more stable and desirable. Such conventional optional ingredients are well known to those skilled in the art, e.g., preservatives such as benzyl alcohol, methyl paraben, propyl paraben and imidazolidinyl urea; betaine surfactants such as lauryl betaine in an amount up to about equal to the amount of amidobetaine; thickeners and viscosity and modifiers such as a diethanolamide of a long chain fatty acid (e.g., coconut diethanol amide), sodium chloride, sodium sulfate, methylcellulose, polyvinyl alcohol, and ethyl alcohol; suspending agents such as hydrogenated castor oil; opacifiers such as ethylene glycol distearate; perfumes; dyes; and, sequestering agents such as disodium ethylenediamine tetraacetate. Such agents, except for the betaine surfactants, generally are used individually at a level of from 0.01% to 10%.

Method of manufacture

The shampoos of the present invention may be made in a variety of ways. A preferred method is set forth in Example 1.

Industrial applicability

The present compositions are used in a conventional manner for cleaning hair. From 0.1 g to 10 g of the composition is applied to hair that has been wetted, generally with water, worked through the hair and then rinsed out.

The following Examples further describe and demonstrate the preferred embodiments within the scope of the present invention. The Examples are given solely for the purpose of illustration and are not to be construed as limitations of the present invention.

Example 1

The following composition was prepared and is representative of the present invention (all %'s are on a 100% active basis):

| Component | | Wt.% |
|---|---|---|
| Mirataine (RTM) BB[1] | | 18.0 |
| Variquat (RTM) E228[2] | | 4.0 |
| Citric Acid | | 4.0 |
| Clindrol (RTM) Superamide[3] 100 CG | | 4.5 |
| Water, perfume, dye and Preservative ph = 3.1 | q.s. | 100.00% |

[1]Lauramidopropyl betaine supplied by Miranol Chemical Company
[2]Cetrimonium chloride supplied by Sherex Chemical Company.
[3]Cocamide diethanol amide supplied by Clintwood Chemical Company.

Preparation of Example 1

The water, betaine and quaternary ammonium compound are mixed together with agitation and heat. Citric acid is then added. When the temperature reaches about 65.5°C, the cocamide DEA is added and the temperature is increased to the 65.5°C—71.1°C range. The batch is kept at 65.5°C—71.1°C until it is clear. The remaining ingredients are then added.

Example II

The following is another composition of the present invention:

| Component | | Wt.% |
|---|---|---|
| Aerosol 30[1] | | 18.0 |
| Variquat (RTM) E228 | | 4.0 |
| Citric Acid | | 4.0 |
| Clindrol (RTM) Superamide 100 CG | | 4.0 |
| Water, perfume, dye and Preservative pH = 2.9 | q.s. | 100.00% |

[1]Cocamidopropyl betaine supplied by American Cyanamid.

Example III

The following is another composition of the present invention:

| Component | | Wt.% |
|---|---|---|
| Mirataine (RTM) BB | | 18.0 |
| Adogen (RTM) 470 DE[1] | | 2.0 |
| Variquat (RTM) E228 | | 2.0 |
| Citric Acid | | 4.0 |
| Clindrol (RTM) Superamide 100 CG | | 2.0 |
| Water, perfume, dye and Preservative pH = 3.1 | q.s | 100.00% |

[1]Ditallowdimonium chloride supplied by Sherex Chemical Company.

Example IV

The following composition was prepared and is representative of the present invention (all %'s are on a 100% active basis)]

| Component | | Wt.% |
|---|---|---|
| Lexaine (RTM) LM[1] | | 18.0 |
| Adogen (RTM) 470DE | | 4.0 |
| Citric Acid | | 4.0 |
| Crotein (RTM) Q[2] | | 1.0 |
| Clindrol (RTM) Superamide 100CG | | 4.0 |
| Ethylene Glycol Distearate | | 1.0 |
| Water, perfume, dye and Preserative pH = 3.0 | q.s. | 100.00% |

[1]Lauramidopropyl betaine supplied by Inolex Chemical Company.
[2]Steartrimonium hydrolized animal protein supplied by Croda, Inc.

Preparation of Example

The betaine, citric acid, and quaternary ammonium compound are mixed together with agitation at room temperature. In a separate vessel, the protein compound is dissolved in room temperature water to make a 20% active solution. In a third vessel, amide, water and ethylene glycol distearate are combined and heated to 71.1°C. All mixtures are agitated until clear solutions are formed. The hot premix is then slowly added to the betaine main mix with high agitation. This cristallizes the ethylene glycol distearate out into small crystals which give the mixture a pearlescent appearance. The protein premix is then added with the remaining ingredients.

Example V

To determine the ability of amido betaine/ammonium compound compositions at pH = 3 to emulsify sebum better than the same composition at pH = 6, the following study was conducted.

Two aqueous solutions were prepared containing 18% cocamidopropyl betaine and 4% tailowdiammonium chloride. One solution was adjusted to pH = 3 while the other had a pH of 6.

These solutions with distilled water to $\frac{1}{8}$ of their original concentration. An aliquot of about 125 grams of each diluted composition was placed at 37.7°C for at least two hours. A sample of each dilute composition was also placed at 100°C until constant weight was obtained.

Artifical sebum, formulated to closely match real sebum, was heated to 37.7°C and kept at that temperature. This heated sebum in an amount of 5 grams was added as a top layer to each of the aliquots which were in separatory funnels.

The separatory funnels were then mounted on a platform capable of rotating at 60 rpm. The funnels were rotated for 960 revolutions after which time the solutions were allowed to settle for $2\frac{1}{2}$ minutes. A 30 g sample of each solution was placed at 100°C until constant weight was obtained.

The net change in mass per gram of betaine surfactant was determined. For the composition of this invention:

$$\text{Fraction of solids in initial dilution} = \frac{\text{solids content found}}{\text{weight of sample}} = \frac{0.57}{16.19} = 0.0352 = Z$$

$$\text{Fraction of solids which is the betaine surfactant} = \frac{\text{(wt. of betaine solution (fraction betaine)}}{\text{[(wt. of the betaine solution) (fraction betaine + fraction salt impurity) + (wt. of ammonium solution) (fraction ammonium) + (amount of acid)]}}$$

$$= \frac{600(0.3096)}{600(0.3096 + 0.0466) + (51.0)\,(.784) + 40} = 0.6325 = Z'$$

$$\text{Fraction of solids in final composition} = \frac{\text{solids content found}}{\text{weight of sample}} = \frac{1.29}{23.16} = 0.0557 = f$$

Net change in mass upon agitation = δ

Z (weight of aliquot (W)) + δ = f (wt. of aliquot + δ)

$$\delta = \frac{(Z - f)W}{f - 1} = \frac{0.0352—0.0057}{0.0557 - 1}(125.53) = 2.7242$$

Net change in mass per gram of betaine surfactant = y

$$y = \frac{\delta}{ZWZ'} = \frac{2.7242}{(0.0352)\ (125.53)\ (06325)} = 0.97$$

A similar calculation for the pH = 6 composition showed that there was only 0.20 grams of change in mass per gram of betaine surfactant.

**Claims**

1. A shampoo composition free of anionic surfactant and comprising:

(A) from 0.5% to 10% of an ammonium compound having the formula

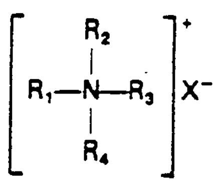

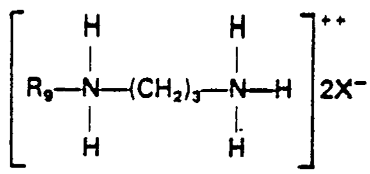

wherein $R_1$ is hydrogen, an aliphatic group of from 1 to 22 carbon atoms, or an aryl or alkaryl group having 6 to 20 carbon atoms; $R_2$ is an aliphatic group having from 12 to 22 carbon atoms; $R_3$ and $R_4$ are each alkyl groups having from 1 to 3 carbon atoms; $R_9$ is an aliphatic group having 16 to 22 carbon atoms; and X is an anion selected from halogen, acetate, phosphate, nitrate and methyl sulfate radicals, or an imidazolinium salt having the formula

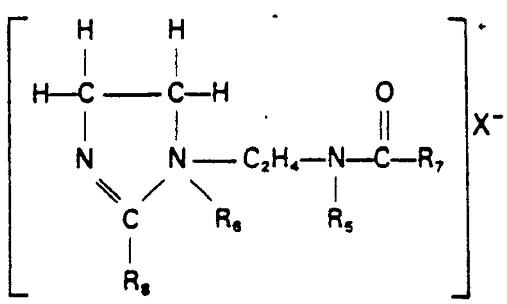

wherein $R_6$ is an alkyl group containing from 1 to 4 carbon atoms; $R_5$ is an alkyl group containing from 1 to 4 carbon atoms or a hydrogen atom; $R_8$ is an alkyl group containing from 1 to 22 carbon atoms, or a hydrogen atom; $R_7$ is an alkyl group containing from 8 to 22 carbon atoms; and X is anion as defined above,

(B) from 5% to 70% of a $C_8$—$C_{18}$ alkylamido betaine surfactant; and

(C) water,

characterized in that the pH of said composition is in the range of 2.0 to 4.0, being maintained within said range by means of an acid buffering agent.

2. A shampoo composition according to Claim 1 wherein the ammonium or imidazolinium compound is present at a level of 1.0% to 6.0%.

3. A shampoo composition according to Claim 1 or 2 wherein the alkylamido betaine is present at a level of from 10% to 25%.

4. A shampoo composition according to any of Claims 1 to 3 wherein the acid buffering agent used to maintain a pH of from 2.0 to 4.0 is selected from citric acid, phosphoric acid, phthalic acid, glycine, and mixtures thereof.

5. A shampoo composition according to any of Claims 1 to 4 wherein the ammonium compound is a dialkyldimethylammonium salt.

6. A shampoo composition according to any of Claims 1 to 5 wherein the alkylamido betaine is an alkylamidopropyl betaine.

7. A shampoo composition according to any of Claims 1 to 6 additionally comprising a diethanolamide of a long chain fatty acid.

8. A shampoo composition according to any of Claims 1 to 7 wherein the ammonium compound is ditallowdimethylammonium chloride.

9. A shampoo composition according to any of Claims 1 to 8 wherein the alkylamido betaine is cocoamidopropyl betaine.

10. A method of cleaning hair comprising:

(a) applying from 0.1 g to 10 g of a composition according to any of Claims 1 to 9 to hair that has been wetted;

(b) working said composition through said hair; and

(c) rinsing said composition from said hair.

**Patentansprüche**

1. Ein von anionischen grenzflächenaktiven Mitteln freies Haarwaschmittel, enthaltend

(A) 0,5% bis 10% einer Ammoniumverbindung der Formel

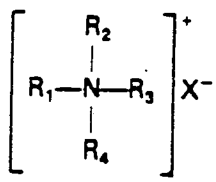

oder

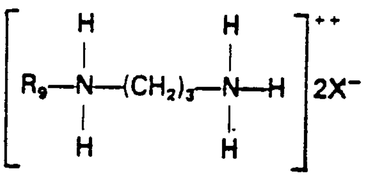

worin $R_1$ Wasserstoff, eine aliphatische Gruppe mit 1 bis 22 Kohlenstoffatomen oder eine Aryl- oder Alkarylgruppe mit 6 bis 20 Kohlenstoffatomen ist; $R_2$ eine aliphatische Gruppe mit 12 bis 22 Kohlenstoffatomen ist; $R_3$ und $R_4$ jeweils Alkylgruppen mit 1 bis 3 Kohlenstoffatomen sind; $R_9$ eine aliphatische Gruppe mit 16 bis 22 Kohlenstoffatomen ist; und X ein Anion ist, das aus Halogen-, Acetat-, Phosphat-, Nitrat- und Methylsulfatresten ausgewählt ist; oder eines Imidazoliniumsalzes der Formel

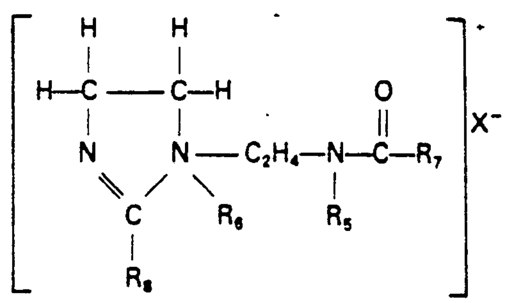

worin $R_6$ eine 1 bis 4 Kohlenstoffatome enthaltende Alkylgruppe ist; $R_5$ eine 1 bis 4 Kohlenstoffatome enthaltende Alkylgruppe oder ein Wasserstoffatom ist; $R_8$ eine 1 bis 22 Kohlenstoffatome enthaltende Alkylgruppe oder ein Wasserstoffatom ist; $R_7$ eine 8 bis 22 Kohlenstoffatome enthaltende Alkylgruppe ist; und X ein wie oben definiertes Anion ist,

(B) 5% bis 70% eines $C_8$—$C_{18}$-Alkylamidobetain-grenzflächenaktiven Mittels; und

(C) Wasser,

dadurch gekennzeichnet, daß der pH des genannten Mittels im Bereich von 2,0 bis 4,0 liegt, und innerhalb des genannten Bereiches durch ein saures Puffermittel aufreicht erhalten wird.

2. Ein Haarwaschmittel nach Anspruch 1, in dem die Ammonium- oder Imidazoliniumverbindung in einer Menge von 1,0% bis 6,0% vorliegt.

3. Ein Haarwaschmittel nach Anspruch 1 oder 2, in dem das Alkylamidobetain in einer Menge von 10% bis 25% vorliegt.

4. Ein Haarwaschmittel nach einem der Ansprüche 1 bis 3, in dem das zur Aufrechterhaltung eines pH-Wertes von 2,0 bis 4,0 verwendete Mittel aus Zitronensäure, Phosphorsäure, Phthalsäure, Glycin, und Mischungen davon, ausgewählt ist.

5. Ein Haarwaschmittel nach einem der Ansprüche 1 bis 4, in dem die Ammoniumverbindung ein Dialkyldimethylammoniumsalz ist.

6. Ein Haarwaschmittel nach einem der Ansprüche 1 bis 5, in dem das Alkylamidobetain ein Alkylamidopropylbetain ist.

7. Ein Haarwaschmittel nach einem der Ansprüche 1 bis 6, welches zusätzlich ein Diethanolamid einer langkettigen Fettsäure enthält.

8. Ein Haarwaschmittel nach einem der Ansprüche 1 bis 7, in dem die Ammoniumverbindung Ditalgdimethylammoniumchlorid ist.

9. Ein Haarwaschmittel nach einem der Ansprüche 1 bis 8, in dem das Alkylamidobetain Kokosnußfettsäureamidopropylbetain ist.

10. Ein Verfahren zur Haarreinigung, umfassend:

(a) das Aufbringen von 0,1 g bis 10 g eines Mittels nach einem der Ansprüche 1 bis 9 auf Haar, das befeuchtet worden ist;

(b) das Durcharbeiten des genannten Haares mit dem genannten Mittel; und

(c) das Abspülen des genannten Mittels aus dem genannten Haar.

**Revendications**

1. Composition de shampooing sans tensioactif anionique et comprenant

(A) de 0,5% à 10% d'un composé d'ammonium répondant à la formule

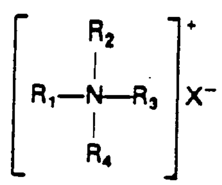

ou

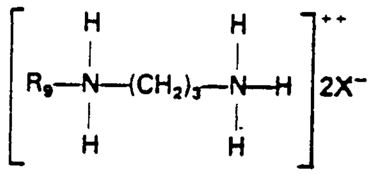

dans laquelle $R_1$ est un hydrogène, un groupe aliphatique de 1 à 22 atomes de carbone, ou un groupe aryle ou alkylaryle ayant 6 à 22 atomes de carbone, ou un groupe aryle ou alkylaryle ayant 6 à 20 atomes de carbone; $R_2$ est un groupe aliphatique ayant de 12 à 22 atomes de carbone; $R_3$ et $R_4$ sont chacun des groupes alkyle ayant de 1 à 3 atomes de carbone; $R_9$ est un groupe aliphatique ayant 16 à 22 atomes de carbone; et X est un anion choisi parmi les radicaux halogène, acétate, phosphate, nitrate et sulfate de méthyle, ou un sel imidazolinium répondant à la formule

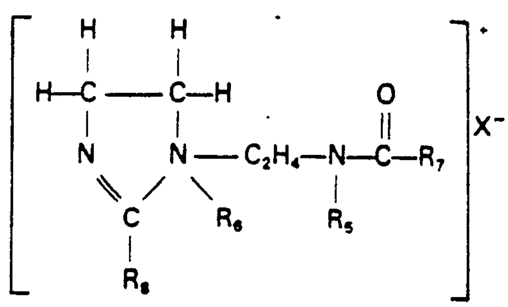

dans laquelle $R_6$ est un groupe alkyle contenant de 1 à 4 atomes de carbone $R_5$ est un groupe alkyle contenant de 1 à 4 atomes de carbone ou un atome d'hydrogène; $R_8$ est un groupe alkyle contenant de 1 à 22 atomes de carbone, ou un atome d'hydrogène; $R_7$ est un groupe alkyle contenant de 8 à 22 atomes de carbone; et X est un anion tel que défini ci-dessus.

(B) de 5% à 70% d'un tensioactif de type alkyl(en $C_8$—$C_{18}$)amidobétaïne; et

(C) de l'eau,

caractérisée en ce que le pH de la dite composition s'échelonne de 2,0 à 4,0, en étant maintenu dans cet intervalle au moyen d'un agent tampon acide.

2. Composition de shampooing selon la revendication 1, dans laquelle le composé d'ammonium ou d'imidazolinium est présent en proportion de 1,0% à 6,0%.

3. Composition de shampooing selon la revendication 1 ou 2, dans laquelle l'alkylamidobétaïne est présente en proportion de 10% à 25%.

4. Composition de shampooing selon l'une quelconque des revendications 1 à 3, dans laquelle l'agent

tampon acide utilisé pour maintenir un pH de 2,0 à 4,0 est choisi parmi l'acide citrique, l'acide phosphorique, l'acide phtalique, la glycine, et leurs mélanges.

5. Composition de shampooing selon l'une quelconque des revendications 1 à 4, dans laquelle le composé d'ammonium est un sel de dialkyldiméthylammonium.

6. Composition de shampooing selon l'une quelconque des revendications 1 à 5, dans laquelle l'alkylamidobétaïne est une alkylamidopropylbétaine.

7. Composition de shampooing selon l'une quelconque des revendications 1 à 6, comprenant en outre un diéthanolamide d'un acide gras à chaîne longue.

8. Composition de shampooing selon l'une quelconque des revendications 1 à 7, dans laquelle le composé d'ammonium est un chlorure de disuifdiméthylammonium.

9. Composition de shampooing selon l'une quelconque des revendications 1 à 8, dans laquelle l'alkylamidobétaïne est l'amido(de coco)propylbétaïne.

10. Procédé de lavage des cheveux comprenant les étapes qui consistent à:

(a) appliquer de 0.1 g à 1.0 g d'une composition selon l'une quelconque des revendications 1 à 9 aux cheveux qui ont été mouillés;

(b) faire pénétrer la dite composition dans les dits cheveux; et

(c) éliminer la dite composition des dits cheveux par rinçage.